# FASCICULE DE BREVET EUROPEEN

(11) **EP 4 090 248 B1**
(45) Date de publication et mention de la délivrance du brevet: **20.11.2024**
(21) Numéro de dépôt: 21700859.8
(22) Date de dépôt: 13.01.2021
(51) Int. Cl.: A61B 6/10

(54) **PARAVENT DE PROTECTION CONTRE LES ÉMISSIONS DE RAYONNEMENTS IONISANTS**
SCHUTZSCHILD GEGEN AUSSTRAHLUNG IONISIERENDER STRAHLUNG
PROTECTION SHIELD AGAINST IONIZATION RADIATION EMISSION

(30) Priorité: 16.01.2020 FR 2000403
(43) Date de publication de la demande: 23.11.2022
(73) Titulaire: Lemer Pax, 44240 La Chapelle-sur-Erdre (FR)
(72) Inventeur: LEMER, Pierre-Marie, 44100 Nantes (FR)
(74) Mandataire: Jacobacci Coralis Harle
(86) Numéro de dépôt international: PCT/EP2021/050531
(87) Numéro de publication internationale: WO 2021/144289

(56) Documents cités:
- WO-A1-2018/109380
- WO-A2-2009/156660

## Description

### Domaine technique de l'invention

La présente invention concerne de manière générale le domaine des équipements de protection contre les rayonnements ionisants.
Elle concerne plus particulièrement les paravents radio-protecteurs qui sont utilisés en milieu médical ou autre, pour protéger un opérateur à l'encontre des émissions de rayonnements ionisants, par exemple les rayons X. Elle concerne également un équipement en forme de housse pour l'habillage par recouvrement de tels paravents, en vue de leur utilisation dans une ambiance protégée stérile.

### Etat de la technique

Dans le cadre de certains examens ou interventions, on soumet les patients à des rayonnements ionisants, type rayons X notamment, utilisés dans un but de contrôle, de diagnostic ou de traitement.

C'est le cas en particulier pour des interventions du genre cathétérisme, pose de pacemakers, examens vasculaires, neurologiques ou urologiques, CRM (Cardiac Rhythm Management), CRT (Cardiac Resynchronization Therapy) ou encore lors de la mise en oeuvre de techniques de fluoroscopie.

En particulier, la fluoroscopie est une technique d'imagerie qui consiste à utiliser les rayons X pour obtenir des images en temps réel d'un objet.

Dans le domaine médical, son application permet la visualisation des structures et fonctions des organes internes d'un patient, comme par exemple le battement cardiaque ou le passage du sang dans les vaisseaux sanguins. Cette technique est utilisée pour le diagnostic ainsi que la thérapie ; et elle est présente dans les domaines interventionnels notamment de la radiologie, cardiologie, neurologie, électrophysiologie, radiologie périphérique vasculaire, pédiatrie interventionnelle etc.

Les salles destinées à ces spécialités sont équipées d'appareils de fluoroscopie (encore appelés C-arm) qui se présentent sous la forme générale d'un caisson technique mobile se prolongeant par un grand arceau dont une extrémité comporte un appareil émetteur de rayon X et dont l'autre extrémité est munie d'un détecteur.

Dans les salles équipées, des cathéters et sondes sont introduits par une voie d'accès (généralement l'artère fémorale ou radiale) dans un but de diagnostic ou thérapeutique. Le réseau vasculaire est visualisé grâce à l'utilisation des rayons X, souvent couplée à une injection de produit(s) de contraste.

Ces appareils de fluoroscopie occupent une place importante autour de la table d'examen et leur positionnement est fréquemment modifié selon la zone corporelle du patient à inspecter ou à traiter.

Certains types d'interventions nécessitent l'assistance de techniques d'échographie, en particulier pour visualiser les parties du coeur qui sont transparentes aux rayons X, donc non visibles sur les écrans de fluoroscopie.

Cette technologie utilisant les ultrasons est par exemple mise en oeuvre pour la pose d'agrafe(s) sur la valve mitrale quand celle-ci ne se referme pas suffisamment bien et n'est plus suffisamment étanche.

De telles techniques d'échographie transoesophagienne (ETO) permettent de visualiser la cavité intracardiaque et en détail la morphologie et les anomalies de l'oreillette gauche et de la paroi du ventricule postérieur. Elles utilisent une sonde de forme tubulaire qui est introduite dans le pharynx puis dans l'oesophage, le transducteur face au côté droit du coeur du patient. Un boitier de commande de la sonde est placé en amont de celle-ci afin de permettre au praticien de réaliser les différents réglages souhaités.

En amont, le boitier de commande de sonde est connecté à une console comprenant notamment un écran de visualisation de l'image cardiaque.

On comprend qu'il est important de protéger correctement les opérateurs (médecins, chirurgiens, techniciens, infirmières ou autre) contre les rayonnements ionisants émis, (de type primaires, provenant directement de l'émetteur, ou de type secondaires : réfléchis par les équipements et issus du patient lui-même), sous peine de les exposer à des doses importantes, cumulées dans le temps, susceptibles d'engendrer des pathologies diverses (nécroses des membres supérieurs, tumeurs cérébrales, cataractes, radiodermites etc.).

Il existe pour cela des structures de protection consistant en des vêtements du genre blouses, chasubles, tabliers en matériau radio-protecteur, protège-thyroïde, lunettes..., mais qui ne couvrent pas toujours la totalité du corps et dont le poids important nuit au confort de l'opérateur, limite ses capacités de mouvements et entraîne une fatigue rapide. De plus leur équivalence de protection de plomb est de 0,5mm d'épaisseur maximum.

Il existe encore des écrans ou paravents constitués de panneaux ou d'assemblages de panneaux en matériau radio-protecteur approprié, suspendus à un support adapté ou posés sur le sol, soit directement, soit par l'intermédiaire d'un piètement roulant.

De telles structures de paravents radio-protecteurs sont décrites dans les documents WO-2018/109380, US-2012/0049093, US-2006/0076522, FR-2 915 868, WO-2009/156660, ou encore US-3 308 297.

Cependant, les structures de paravent connues jusqu'à présent ne sont pas complètement adaptées à la mise en oeuvre des techniques d'échographie précitées, en particulier du fait de la présence de matériels ou d'équipements qui doivent être manipulés lors de telles interventions.

### Présentation de l'invention

Afin de remédier à l'inconvénient précité de l'état de la technique, la présente invention propose un paravent de radioprotection pour assurer la protection d'un opérateur contre les émissions de rayonnements ionisants du type rayons X ou autres, lequel paravent comprend une paroi frontale réalisée au moins en partie en un matériau radio-protecteur, laquelle paroi frontale comprend une face intérieure de paroi frontale, orientée vers l'espace de positionnement de l'opérateur, une face extérieure de paroi frontale, opposée à ladite face intérieure de paroi frontale, une partie supérieure, une partie inférieure et une partie intermédiaire située entre lesdites parties supérieure et inférieure, laquelle partie supérieure comprend au moins un panneau transparent et laquelle partie intermédiaire comprend une structure de sas, adaptée au passage de matériel(s) et/ou des bras dudit opérateur,
ce paravent de radioprotection étant caractérisé par le fait que ladite structure de sas comprend une ouverture de sas associée à une structure d'obturation souple, laquelle ouverture de sas comprend une bordure inférieure d'ouverture, horizontale ou approximativement horizontale,
et par le fait qu'il comprend un dispositif support adapté au support de matériel(s) et/ou d'un bras de l'opérateur, lequel dispositif support est disposé au niveau de ladite bordure inférieure d'ouverture, du côté de ladite face intérieure de paroi frontale.

Un tel dispositif support qui s'étend dans l'espace de positionnement de l'opérateur facilite la manipulation de matériels ou d'équipements par cet opérateur, diminuant ainsi sa fatigue dans le temps et augmentant la précision de ses gestes.

D'autres caractéristiques non limitatives et avantageuses du paravent de radioprotection conforme à l'invention, prises individuellement ou selon toutes les combinaisons techniquement possibles, sont les suivantes :
- ledit dispositif support comprend une extrémité proximale située à proximité de ladite bordure inférieure d'ouverture et une extrémité distale opposée, et ladite extrémité proximale dudit dispositif support est montée pivotante autour d'un axe vertical ou approximativement vertical.
- le paravent de radioprotection comprend des moyens de réglage pour permettre un réglage de la position dudit dispositif support sur une partie au moins de la longueur de ladite bordure inférieure d'ouverture.
- lesdits moyens de réglage consistent en une structure de rail ménagée le long de ladite bordure inférieure d'ouverture, coopérant avec une structure de coulisseau solidaire dudit dispositif support, laquelle structure de coulisseau est munie de moyens de verrouillage adaptés pour permettre un verrouillage de sa position sur ladite structure de rail.
- ledit dispositif support se présente sous la forme d'un corps allongé.
- ledit dispositif support se présente sous la forme d'une goulotte à section en U.
- ladite paroi frontale du paravent de radioprotection comprend (a) un panneau extérieur, formant une partie au moins de ladite face extérieure de paroi frontale, lequel panneau extérieur comprend une ouverture de panneau extérieur située au niveau de la partie intermédiaire de paroi frontale, et (b) un panneau intérieur, mobile verticalement, disposé en regard de ladite ouverture de panneau extérieur, du côté de ladite face intérieure de paroi frontale,
   lequel panneau intérieur comprend une ouverture de panneau intérieur, laquelle ouverture de panneau intérieur comprend ladite structure d'obturation souple et comprend ladite bordure inférieure d'ouverture équipée dudit dispositif support,
   laquelle ouverture de panneau extérieur et laquelle ouverture de panneau intérieur forment ensemble ladite ouverture de sas,
   et lequel panneau intérieur est monté mobile verticalement par rapport audit panneau extérieur de manière à permettre d'adapter le niveau de hauteur de ladite ouverture de sas.
- ledit panneau intérieur est monté mobile le long de glissières verticales ménagées sur ledit panneau extérieur, en association avec des moyens compensateurs de poids, lequel panneau intérieur est muni d'une poignée de manoeuvre et comprend un doigt d'indexation coopérant avec une pluralité d'orifices d'indexation prévus sur ledit panneau extérieur, pour assurer le verrouillage de sa position en hauteur.
- ladite ouverture de panneau intérieur et ladite ouverture de panneau extérieur comprennent chacune deux parties d'ouverture juxtaposées horizontalement et séparées par un montant vertical.
- la structure d'obturation souple consiste en une juxtaposition d'une pluralité de bandes verticales réalisées en matériau radio-protecteur.
- ladite paroi frontale s'étend dans un plan vertical ou approximativement vertical et comprend deux bordures latérales, au moins l'une desdites bordures latérales de ladite paroi frontale se prolongeant par une paroi latérale qui s'étend dans un plan vertical ou approximativement vertical, selon un angle compris entre 90° et 140° par rapport au plan de ladite paroi frontale, vu du côté de ladite face intérieure de paroi frontale, laquelle ou lesquelles parois latérales comportent une bordure latérale externe équipée d'un bavette souple en matériau radio-protecteur.
- ladite paroi frontale s'étend dans un plan vertical ou approximativement vertical et comporte deux bordures latérales se prolongeant chacune par une paroi latérale, lesquelles parois latérales s'étendent chacune dans un plan vertical ou approximativement vertical, selon un angle compris entre 90° et 140° par rapport au plan de ladite paroi frontale, vu du côté de ladite face intérieure de paroi frontale,
   et ladite structure de sas s'étend sur toute la largeur de ladite paroi frontale et se prolonge sur une partie de la largeur de chacune desdites parois latérales.
- le paravent de radioprotection comprend un écran monté sur un support d'écran fixé sur ladite face intérieure de paroi frontale ou, le cas échéant, sur une face intérieure de l'une de ses parois latérales.
- le paravent de radioprotection comprend un piètement muni de roues d'appui au sol, et la partie inférieure de sa paroi frontale, et le cas échéant une partie inférieure de ses parois latérales, comporte un panneau en matériau radio-protecteur souple.

La présente invention concerne encore un équipement en forme de housse destiné à recouvrir au moins une partie de la hauteur d'un paravent de radioprotection tel que défini ci-dessus comprenant :
(a) au moins un panneau souple adapté pour venir recouvrir au moins partiellement ladite paroi frontale et le cas échéant au moins partiellement ladite ou lesdites parois latérales, ledit au moins un panneau souple comportant au moins une partie transparente destinée à venir se positionner en regard du panneau transparent de la partie supérieure de ladite paroi frontale, et des moyens de fixation sur ladite paroi frontale et/ou, le cas échéant, sur ladite ou lesdites parois latérales,
(b) au moins une structure souple de sas, adaptée pour venir recouvrir au moins partiellement la structure d'obturation souple associée à ladite ouverture de sas de ladite structure de sas, laquelle au moins une structure souple de sas se présente sous la forme d'une poche souple munie d'une ouverture, laquelle poche souple est adaptée pour venir recouvrir ladite structure d'obturation souple par engagement de la bordure inférieure de cette dernière dans ladite ouverture de ladite poche souple, laquelle structure souple de sas est munie de moyens pour sa fixation sur ladite structure de sas, et
(c) une poche souple de dispositif support, pour le recouvrement dudit dispositif support adapté au support de matériel(s) et/ou d'un bras de l'opérateur, laquelle poche souple de dispositif support comprend une ouverture de poche pour son positionnement sur ledit dispositif support, laquelle poche souple de dispositif support est munie de moyens pour sa fixation sur ledit dispositif support.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée en référence aux dessins qui illustrent des formes, non limitatives, de réalisation de l'invention et où :
[Fig. 1] représente un paravent radio-protecteur conforme à l'invention, vu en perspective du côté intérieur, c'est-à-dire du côté de l'espace de positionnement de l'opérateur ;
[Fig. 2] est une vue de face, côté intérieur, du paravent radio-protecteur de la figure 1 ;
[Fig. 3] est une vue de côté du paravent radio-protecteur illustré sur les figures 1 et 2 ;
[Fig. 4] est une vue de face, côté extérieur (à l'opposé de l'espace de positionnement de l'opérateur), du paravent radio-protecteur illustré sur les figures 1 à 3 ;
[Fig. 5] est une vue de dessus du paravent radio-protecteur illustré sur les figures 1 à 4.

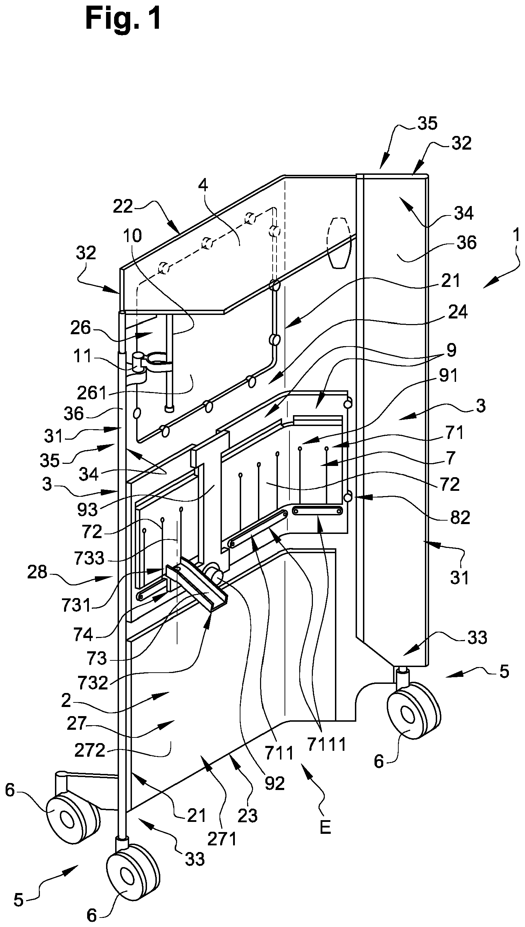

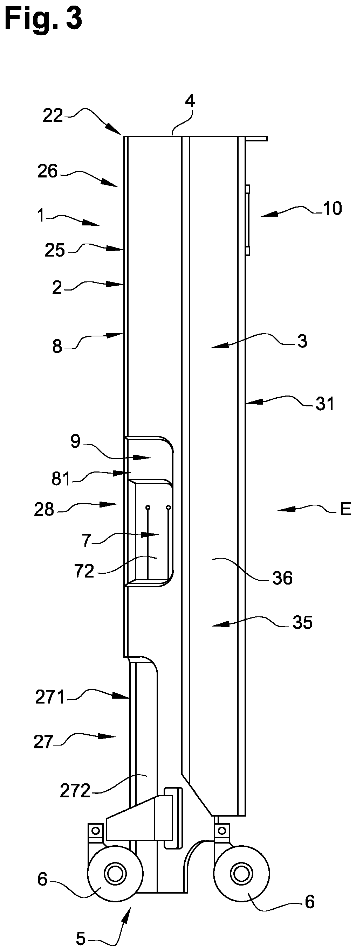

Le paravent 1 de radioprotection illustré sur les figures 1 à 5 est adapté pour assurer la protection d'un opérateur contre les rayonnements ionisants émis par une source de rayonnements ionisants, par exemple des rayons X émis par un appareil de fluoroscopie du type C-arm, dans une salle d'opération hospitalière.

Pour cela les différentes parties constitutives de ce paravent 1 radio-protecteur sont réalisées en matériau(x) radio-protecteur(s) ayant une équivalence plomb adaptée, d'au minimum 0,2mm, selon les parties constitutives concernées. Par exemple, pour toutes les parties constitutives, cette équivalence plomb est comprise entre 0,5mm et 3mm.

Plus particulièrement, le paravent 1 de radioprotection est constitué d'une ossature métallique, par exemple en acier, qui est associée à différents panneaux en matériau radio-protecteur approprié, tels que des panneaux transparents ou des panneaux souples.

Comme illustré sur les figures 1 à 5, ce paravent 1 radio-protecteur se présente sous la forme d'une cabine mobile comprenant une paroi frontale 2 prolongée sur les côtés par des parois latérales 3, et en partie supérieure par une structure de plafond 4, l'ensemble étant monté sur un piètement 5 muni de roues 6 d'appui au sol.

La paroi frontale 2 s'étend dans un plan vertical ou approximativement vertical.

Cette paroi frontale 2, de forme générale rectangulaire, est délimitée par deux bordures latérales 21, une bordure supérieure 22 et une bordure inférieure 23. Elle comprend une face intérieure de paroi frontale 24, orientée vers l'espace de positionnement de l'opérateur E, et une face extérieure de paroi frontale 25, opposée à ladite face intérieure de paroi frontale 24.

Les parois latérales 3 prolongent chacune l'une des bordures latérales 21 de la paroi frontale 2.

Chaque paroi latérale 3 est de forme générale rectangulaire et s'étend sur toute ou pratiquement toute la hauteur de la bordure latérale 21 attenante de la paroi frontale 2.

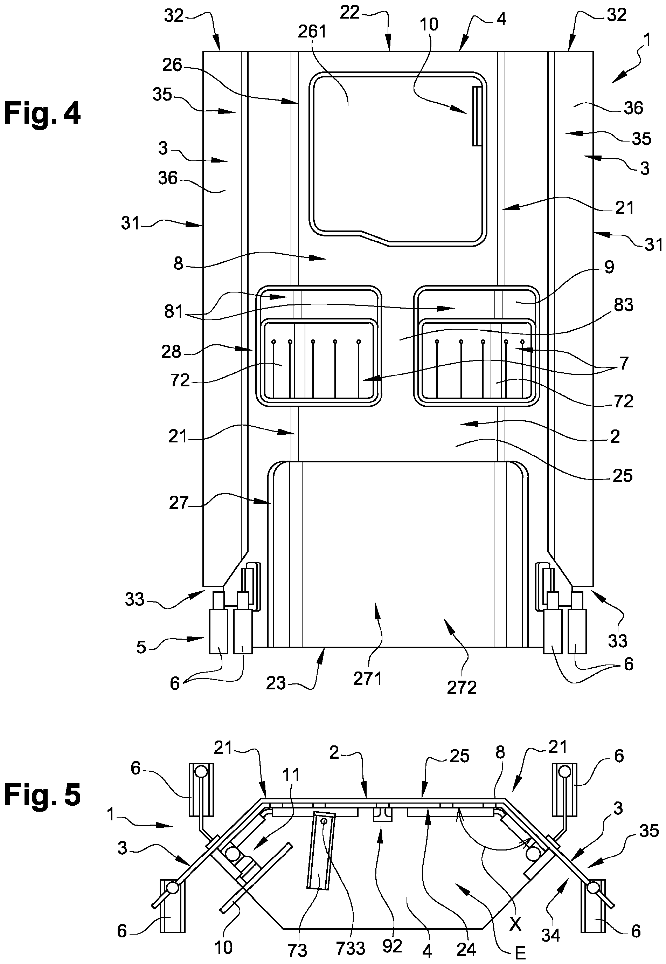

Chacune de ces parois latérales 3 s'étend dans un plan vertical ou approximativement vertical, et cela de manière inclinée selon un angle X (figure 5) compris entre 90° et 140° par rapport au plan de la paroi frontale 2, vu du côté de la face intérieure de paroi frontale 24.

De préférence cet angle X entre la face intérieure de paroi frontale 24 et chaque paroi latérale 3 est compris entre 110° et 120°.

De plus, chacune de ces parois latérales 3 est délimitée par une bordure latérale externe 31, une bordure supérieure 32 et une bordure inférieure 33, et comprend une face intérieure de paroi latérale 34, orientée vers l'espace de positionnement de l'opérateur E, ainsi qu'une face extérieure de paroi latérale 35, opposée à ladite face intérieure de paroi latérale 34.

La bordure latérale externe 31 des parois latérales 3 est équipée d'une bavette souple 36 en matériau radio-protecteur. De telles bavettes souples 36 latérales permettent d'absorber les chocs et elles limitent les risques de détérioration du paravent 1 ou des matériels environnants en cas de contact avec ces matériels.

Les bavettes souples 36 consistent par exemple en une feuille de matériau souple radio-protecteur recouvert d'un tissu de protection résistant aux projections liquides, cette feuille étant avantageusement repliée sur elle-même pour former une double épaisseur.

La partie supérieure 26 de la paroi frontale 2 comprend un panneau transparent 261 pour permettre à l'opérateur d'accéder visuellement de l'autre côté du paravent 1. Par exemple, ce panneau transparent 261 peut être réalisé en verre au plomb ou en matière plastique chargée de matériau radio-protecteur.

Dans le mode de réalisation illustré, le panneau transparent 261 s'étend sur une partie de la largeur de la paroi frontale 2. Dans une variante de réalisation, ce panneau transparent 261 peut occuper toute la largeur de la paroi frontale 2 ; et éventuellement il peut aussi s'étendre latéralement pour occuper une partie de la surface des parois latérales 3.

De son côté, la partie inférieure 27 de la paroi frontale 2 comprend une ouverture 271 comblée par un panneau 272 en matériau radio-protecteur souple. Ce panneau souple 272 est capable de se déformer sous le contact d'un équipement (par exemple le C-arm de fluoroscopie), pour éviter sa dégradation, tout en constituant une barrière efficace contre les rayonnements ionisants.

Ici, ce panneau 271 en matériau radio-protecteur souple occupe toute la largeur de la paroi frontale 2 et également une partie de la surface des parois latérales 3. Dans une variante de réalisation le panneau 271 en matériau radio-protecteur souple peut s'étendre sur une partie seulement de la largeur de la paroi frontale 2.

Le panneau souple 272 peut par exemple être constitué d'un assemblage multi-feuilles de matériau souple radio-protecteur recouvert d'un tissu de protection résistant aux projections liquides, le tout formant un rideau souple radio-protecteur.

Entre sa partie supérieure 26 et sa partie inférieure 27, la paroi frontale 2 comprend une partie intermédiaire 28 munie d'une structure de sas 7 permettant le passage de matériel(s) et/ou des bras de l'opérateur de l'autre côté du paravent.

La structure de sas 7 comprend une ouverture de sas 71 associée à une structure d'obturation souple 72. L'ouverture de sas 71 comprend une bordure inférieure d'ouverture 711 et cette bordure inférieure d'ouverture 711 comprend un dispositif support 73 qui est adapté au support de matériel(s) et/ou d'un bras de l'opérateur.

Le dispositif support 73 est disposé du côté de la face intérieure de paroi frontale 24 (c'est-à-dire qu'il s'étend depuis la bordure inférieure d'ouverture 711 dans l'espace de positionnement E de l'opérateur). Il consiste ici en un corps allongé en forme de goulotte à section en U. Il comprend une extrémité proximale 731 fixée au niveau de la bordure inférieure d'ouverture 711 et une extrémité distale 732 opposée, qui s'étend dans l'espace de positionnement E. L'extrémité proximale 731 du dispositif support 73 est montée pivotante à proximité de la bordure inférieure d'ouverture 711, autour d'un axe 733 vertical ou approximativement vertical.

La position du dispositif support 73 peut être réglée sur une partie au moins de la longueur de la bordure inférieure d'ouverture 711.

Pour cela, l'extrémité proximale 731 du dispositif support 73 comprend une structure de coulisseau 74 qui est adaptée pour coopérer avec une structure de rail 7111 ménagée le long de la bordure inférieure d'ouverture 711.

De préférence la structure de coulisseau 74 est munie de moyens de verrouillage 75 adaptés pour permettre un verrouillage de sa position (et donc de la position du dispositif support 73) sur la structure de rail 7111. Ces moyens de verrouillage 75 peuvent par exemple consister en une vis de serrage portée par la structure de coulisseau 74 et dont l'extrémité est agencée pour venir en contact avec la structure de rail 7111, ou encore en un piston avec rappel par ressort.

On comprend qu'un opérateur présent dans l'espace de positionnement E peut utiliser le dispositif support 73 comme support de bras ou de matériel lorsqu'il intervient au travers du sas 7. Par exemple le dispositif support 73 peut servir de support pour le boitier de commande de la sonde du matériel d'échographie intracardiaque manipulé par un opérateur échographiste, en assistance au chirurgien cardiaque.

Le positionnement du dispositif support 73 le long de la structure de rail 7111 est réglé en fonction de la morphologie de l'opérateur échographiste. Et le montage pivotant de ce dispositif support 73 autour de l'axe vertical 733 facilite les mouvements de l'opérateur au cours de son intervention.

La forme générale et la structure du dispositif support 73 peuvent être adaptées selon le matériel/équipement destiné à être supporté et manipulé par l'opérateur.

Plus précisément, la paroi frontale 2 du paravent 1 radio-protecteur comprend :
- (a) un panneau extérieur 8, muni d'une ouverture de panneau extérieur 81 située au niveau de la partie intermédiaire de paroi frontale 28, et
- (b) un panneau intérieur 9, mobile verticalement, disposé en regard de ladite ouverture de panneau extérieur 81, du côté de la face intérieure de paroi frontale 24, lequel panneau intérieur 9 comprend une ouverture de panneau intérieur 91 qui comprend elle-même la structure d'obturation souple 72, ainsi que la bordure inférieure d'ouverture 711 équipée du dispositif support 73.

L'ouverture de panneau extérieur 81 et l'ouverture de panneau intérieur 91 forment ensemble l'ouverture de sas 71 précitée.

L'ouverture de panneau extérieur 81 est plus grande que l'ouverture de panneau intérieur 91 et l'ouverture de sas 71 est définie par le positionnement de l'ouverture de panneau intérieur 91 en regard de l'ouverture de panneau extérieur 81. Les dimensions et le positionnement de l'ouverture de panneau extérieur 81 sont adaptés pour permettre toutes les positions admissibles/souhaitées en hauteur de l'ouverture de panneau intérieur 91.

Le panneau intérieur 9 est monté mobile verticalement par rapport au panneau extérieur 8 de manière à permettre d'adapter le niveau de hauteur de l'ouverture de panneau intérieur 91, et donc de l'ouverture de sas 71.

Pour cela, le panneau intérieur 9 est guidé par des glissières verticales 82 ménagées sur le panneau extérieur 8, sur la face de ce dernier orientée vers l'espace de positionnement E. Ce panneau intérieur 9 est en outre muni d'une poignée de manoeuvre 92 et de moyens d'indexation pour assurer le verrouillage de sa position en hauteur.

Par exemple les moyens d'indexation consistent en un doigt d'indexation escamotable équipant la poignée de manoeuvre 92, coopérant avec une pluralité d'orifices d'indexation (non représentés) prévus sur le panneau extérieur 8. La poignée de manoeuvre 92 peut alors disposer d'une possibilité de mouvement horizontal, perpendiculairement au mouvement du panneau intérieur 9, et être associée à un moyen de rappel en position de verrouillage.

De préférence le paravent radio-protecteur 1 comprend encore des moyens compensateurs de poids, non représentés, qui permettent de compenser le poids du panneau intérieur 9 de sas, afin de faciliter son réglage de position en hauteur. Ces moyens compensateur de poids peuvent consister en un ou plusieurs vérins (par exemple des vérins du type à gaz), interposés entre le panneau extérieur 8 et le panneau intérieur 9.

Comme on peut le voir sur les figures 1, 2 et 4, dans le mode de réalisation illustré, l'ouverture de panneau extérieur 81 et l'ouverture de panneau intérieur 91 comprennent deux parties d'ouverture juxtaposées horizontalement et séparées par un montant vertical, respectivement 83 et 93.

Ces deux montants verticaux 83 et 93 sont disposés en vis-à-vis et ils peuvent servir de support pour le ou les vérins compensateurs de poids précités.

La structure d'obturation souple 72 équipe les deux parties de l'ouverture de panneau intérieur 91, de chaque côté du montant vertical 93.

Cette structure d'obturation souple 72 est agencée pour fermer le plus efficacement possible les deux parties de l'ouverture de panneau intérieur 91, tout en permettant leur écartement pour laisser passer du matériel et/ou les bras de l'opérateur.

La structure d'obturation souple 72 peut consister en une juxtaposition d'une pluralité de bandes verticales réalisées en matériau radio-protecteur ; cette juxtaposition de bandes verticales peut être fixée au niveau de la partie supérieure de chacune des deux parties de l'ouverture de panneau intérieur 91 (par exemple au moyen de profilés métalliques de fixation vissés), et elle s'étend sur toute la largeur et sur toute la hauteur des deux parties de l'ouverture de panneau intérieur 91.

De telles bandes souples peuvent être obtenues à partir d'un panneau souple réalisé en Novashield (marque déposée), qui consiste en un matériau souple radio-protecteur sans plomb.

A titre de variante, les bandes en matériau souple peuvent être remplacées par des bandes formées d'une juxtaposition de maillons rigides en matériau radio-protecteur, reliés les uns aux autres par des articulations.

Comme illustré sur les figures annexées, la structure de sas 7 s'étend sur toute la largeur de la paroi frontale 2 et sur une partie de la largeur de chaque paroi latérale 3. Le panneau intérieur 9 de la structure de sas 7 présente donc ici une forme triédrique constituée d'une juxtaposition de trois faces décalées angulairement les unes par rapport aux autres. La structure de rail 7111 qui porte le dispositif support 73 est ménagée le long de la bordure inférieure d'ouverture 711 des deux parties de l'ouverture de panneau intérieur 91. Cette structure de rail 7111 est constituée de plusieurs tronçons qui suivent la forme triédrique du panneau intérieur 9.

Dans une variante de réalisation, la paroi frontale 2 peut être munie d'une seule paroi latérale 3 ; et encore dans une autre variante de réalisation cette paroi frontale 2 peut ne pas comporter de paroi latérale. La forme générale de la structure de sas 7 est alors adaptée en conséquence.

Selon une autre particularité avantageuse, le paravent radio-protecteur 1 comprend un écran 10 disposé dans l'espace de positionnement de l'opérateur E.

Cet écran 10 est adapté pour afficher des informations utiles à l'opérateur positionné dans l'espace E. Par exemple cet écran 10 peut afficher les paramètres biologiques du patient (rythme cardiaque, température, pression artérielle...), ainsi que les images obtenues par le système de fluoroscopie (C-Arm).

L'écran 10 est monté sur un support d'écran 11 qui est fixé sur la face intérieure de paroi frontale 24 ou sur une face intérieure de paroi latérale 34.

En l'occurrence, dans le mode de réalisation illustré un support d'écran 11 est fixé sur chacune des parois latérales 3, du côté de l'espace de positionnement de l'opérateur E, pour permettre un choix du positionnement le plus judicieux de l'écran 10.

Par exemple, le paravent radio-protecteur 1 selon l'invention peut être réalisé à partir d'une ossature en tôle métallique ayant 12mm d'épaisseur, formant le panneau extérieur 8 de la paroi frontale 2 et une partie des parois latérales 3, laquelle ossature métallique comprend :
- une ouverture supérieure pour le positionnement du panneau transparent 261, rapporté du côté de l'espace de positionnement de l'opérateur E, et fixé par tout moyen approprié, par exemple des pions en matière plastique ;
- l'ouverture de panneau extérieur 81, pour le positionnement en regard du panneau intérieur 9 mobile équipé du dispositif support 73 ;
- l'ouverture inférieure 271 pour le positionnement du panneau 272 en matériau radio-protecteur souple, rapporté du côté de l'espace de positionnement de l'opérateur E, et fixé par tout moyen approprié (par exemple par des profilés métalliques de fixation vissés au niveau de la bordure supérieure de cette ouverture inférieure 271) ;
- les bavettes souples 36 en matériau radio-protecteur rapportées et fixées par tout moyen approprié sur les bordures latérales externes 31 des parois latérales 3 ;
- la structure de plafond 4 formée d'un panneau transparent en matériau radio-protecteur, qui s'étend horizontalement à partir de la bordure supérieure 22 de la paroi frontale 2, et de la bordure supérieure 32 des parois latérales 3, fixée par tout moyen approprié ;
- le piètement 5 muni des roues 6 d'appui au sol ;
- l'écran 10 avec son support d'écran 11.

Lors de son utilisation en salle opératoire, le paravent radio-protecteur 1 est associé à un équipement, en forme de housse stérile, destiné à recouvrir au moins une partie de sa hauteur.

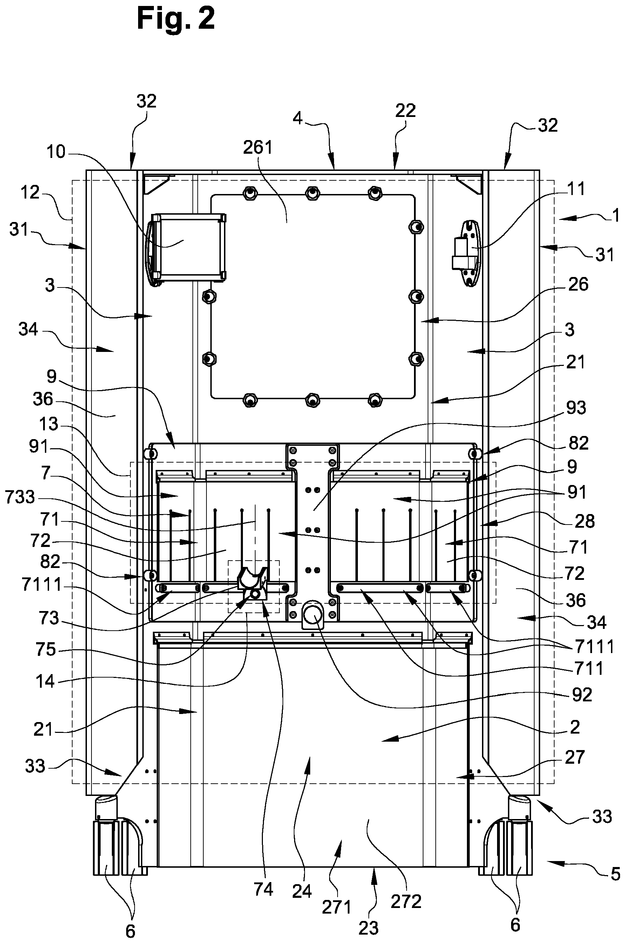

Un tel équipement en forme de housse, représenté schématiquement sur la figure 2, peut comprendre :
- un panneau souple 12 adapté pour venir recouvrir au moins partiellement les faces intérieures et extérieures de la paroi frontale 2 et des parois latérales 3. Ce panneau souple 12 comporte une ouverture au niveau de sa partie destinée à venir se positionner en regard de la structure de sas 7 ; et il comporte une partie transparente destinée à venir se positionner en regard du panneau transparent 261 de la partie supérieure 26 de la paroi frontale 2. Le panneau 12 comporte encore des moyens de fixation pour permettre sa fixation sur la paroi frontale 2 et/ou sur les parois latérales 3. Ces moyens de fixation peuvent par exemple consister en des attaches, des bandes d'adhésif ou encore des bandes d'auto agrippant.
- au moins une structure souple de sas 13, adaptée pour venir recouvrir au moins partiellement la structure d'obturation souple 72 associée à l'ouverture de sas 71 de la structure de sas 7. Cette structure souple de sas 13 peut se présenter sous la forme d'au moins une poche souple munie d'une ouverture, laquelle poche souple est adaptée pour venir recouvrir ladite structure d'obturation souple 72 par engagement de la bordure inférieure de cette dernière dans l'ouverture de ladite poche souple. Cette structure souple de sas 13 est encore munie de moyens pour permettre sa fixation sur la structure de sas 7 (par exemple des attaches, des bandes d'adhésif ou encore des bandes d'auto agrippant).

En l'occurrence, la structure souple de sas 13 peut comporter deux poches souples adaptée chacune pour venir recouvrir l'une des deux parties de structure d'obturation souple 72, de part et d'autre du montant 93.
- une poche souple de dispositif support 14, pour le recouvrement du dispositif support 73, laquelle poche souple de dispositif support 14 comprend une ouverture de poche pour son positionnement sur ledit dispositif support 73. Cette poche souple de dispositif support 14 est munie de moyens pour sa fixation sur le dispositif support 73 (par exemple des attaches, des bandes d'adhésif ou encore des bandes d'auto agrippant).

## Revendications

1. Paravent (1) de radioprotection pour assurer la protection d'un opérateur contre les émissions de rayonnements ionisants du type rayons X ou autres, lequel paravent (1) comprend une paroi frontale (2) réalisée au moins en partie en un matériau radio-protecteur, laquelle paroi frontale (2) comprend une face intérieure de paroi frontale (24), orientée vers un espace de positionnement de l'opérateur (E), une face extérieure de paroi frontale (25), opposée à ladite face intérieure de paroi frontale (24), une partie supérieure (26), une partie inférieure (27) et une partie intermédiaire (28) située entre lesdites parties supérieure (26) et inférieure (27), laquelle partie supérieure (26) comprend au moins un panneau transparent (261) et laquelle partie intermédiaire (28) comprend une structure de sas (7), adaptée au passage de matériel(s) et/ou des bras dudit opérateur,
laquelle structure de sas (7) comprend une ouverture de sas (71) associée à une structure d'obturation souple (72), laquelle ouverture de sas (71) comprend une bordure inférieure d'ouverture (711), horizontale ou approximativement horizontale,
**caractérisé en ce que** ledit paravent (1) comprend un dispositif support (73) adapté au support de matériel(s) et/ou d'un bras de l'opérateur, lequel dispositif support (73) est disposé au niveau de ladite bordure inférieure d'ouverture (711), du côté de ladite face intérieure de paroi frontale (24).

2. Paravent de radioprotection selon la revendication 1, **caractérisé en ce que** ledit dispositif support (73) comprend une extrémité proximale (731) située à proximité de ladite bordure inférieure d'ouverture (711) et une extrémité distale (732) opposée, et **en ce que** ladite extrémité proximale (731) dudit dispositif support (73) est montée pivotante autour d'un axe (733) vertical ou approximativement vertical.

3. Paravent de radioprotection selon l'une quelconque des revendications 1 ou 2, **caractérisé en ce qu'**il comprend des moyens de réglage (74, 7111) pour permettre un réglage de la position dudit dispositif support (73) sur une partie au moins de la longueur de ladite bordure inférieure d'ouverture (711).

4. Paravent de radioprotection selon la revendication 3, **caractérisé en ce que** lesdits moyens de réglage consistent en une structure de rail (7111) ménagée le long de ladite bordure inférieure d'ouverture (711), coopérant avec une structure de coulisseau (74) solidaire dudit dispositif support (73), laquelle structure de coulisseau (74) est munie de moyens de verrouillage adaptés pour permettre un verrouillage de sa position sur ladite structure de rail (7111) .

5. Paravent de radioprotection selon l'une quelconque des revendications 1 à 4, **caractérisé en ce que** ledit dispositif support (73) se présente sous la forme d'un corps allongé.

6. Paravent de radioprotection selon la revendication 5, **caractérisé en ce que** ledit dispositif support (73) se présente sous la forme d'une goulotte à section en U.

7. Paravent de radioprotection selon l'une quelconque des revendications 1 à 6, **caractérisé en ce que** ladite paroi frontale (2) comprend (a) un panneau extérieur (8), formant une partie au moins de ladite face extérieure de paroi frontale (25), lequel panneau extérieur (8) comprend une ouverture de panneau extérieur (81) située au niveau de la partie intermédiaire de paroi frontale (28), et (b) un panneau intérieur (9), mobile verticalement, disposé en regard de ladite ouverture de panneau extérieur (81), du côté de ladite face intérieure de paroi frontale (24),
lequel panneau intérieur (9) comprend une ouverture de panneau intérieur (91), laquelle ouverture de panneau intérieur (91) comprend ladite structure d'obturation souple (72) et comprend ladite bordure inférieure d'ouverture (711) équipée dudit dispositif support (73),
laquelle ouverture de panneau extérieur (81) et laquelle ouverture de panneau intérieur (91) forment ensemble ladite ouverture de sas (71),
et lequel panneau intérieur (9) est monté mobile verticalement par rapport audit panneau extérieur (8) de manière à permettre d'adapter le niveau de hauteur de ladite ouverture de sas (71).

8. Paravent de radioprotection selon la revendication 7, **caractérisé en ce que** ledit panneau intérieur (9) est monté mobile le long de glissières verticales (82) ménagées sur ledit panneau extérieur (8), en association avec des moyens compensateurs de poids, lequel panneau intérieur (9) est muni d'une poignée de manoeuvre (92) et comprend un doigt d'indexation coopérant avec une pluralité d'orifices d'indexation prévus sur ledit panneau extérieur (8), pour assurer le verrouillage de sa position en hauteur.

9. Paravent de radioprotection selon l'une quelconque des revendications 7 ou 8, **caractérisé en ce que** ladite ouverture de panneau intérieur (91) et ladite ouverture de panneau extérieur (81) comprennent chacune deux parties d'ouverture juxtaposées horizontalement et séparées par un montant vertical (83, 93).

10. Paravent de radioprotection selon l'une quelconque des revendications 1 à 9, **caractérisé en ce que** ladite structure d'obturation souple (72) consiste en une juxtaposition d'une pluralité de bandes verticales réalisées en matériau radio-protecteur.

11. Paravent de radioprotection selon l'une quelconque des revendications 1 à 10, **caractérisé en ce que** ladite paroi frontale (2) s'étend dans un plan vertical ou approximativement vertical et comprend deux bordures latérales (21), au moins l'une desdites bordures latérales (21) de ladite paroi frontale (2) se prolongeant par une paroi latérale (3) qui s'étend dans un plan vertical ou approximativement vertical, selon un angle (X) compris entre 90° et 140° par rapport au plan de ladite paroi frontale, vu du côté de ladite face intérieure de paroi frontale (24),
laquelle ou lesquelles parois latérales (3) comportent une bordure latérale externe (31) équipée d'un bavette souple (36) en matériau radio-protecteur.

12. Paravent de radioprotection selon l'une quelconque des revendications 1 à 11, **caractérisé en ce que** ladite paroi frontale (2) s'étend dans un plan vertical ou approximativement vertical et comporte deux bordures latérales (21) se prolongeant chacune par une paroi latérale (3), lesquelles parois latérales (3) s'étendent chacune dans un plan vertical ou approximativement vertical, selon un angle (X) compris entre 90° et 140° par rapport au plan de ladite paroi frontale (2), vu du côté de ladite face intérieure de paroi frontale (24),
et **en ce que** ladite structure de sas (7) s'étend sur toute la largeur de ladite paroi frontale (2) et se prolonge sur une partie de la largeur de chacune desdites parois latérales (3).

13. Paravent de radioprotection selon l'une quelconque des revendications 1 à 12, **caractérisé en ce qu'**il comprend un écran (10) monté sur un support d'écran (11) fixé sur ladite face intérieure de paroi frontale (24) ou, le cas échéant, sur une face intérieure (34) de l'une de ses parois latérales (3).

14. Paravent de radioprotection selon l'une quelconque des revendications 1 à 13, **caractérisé en ce qu'**il comprend un piètement (5) muni de roues d'appui au sol (6), et **en ce que** la partie inférieure (27) de sa paroi frontale (2) et, le cas échéant, une partie inférieure de ses parois latérales (3), comporte un panneau en matériau radio-protecteur souple (271).

15. Équipement en forme de housse destiné à recouvrir au moins une partie de la hauteur d'un paravent de radioprotection (1) selon l'une quelconque des revendication 1 à 14, **caractérisé en ce qu'**il comprend :
- au moins un panneau souple (12) adapté pour venir recouvrir au moins partiellement ladite paroi frontale (2) et le cas échéant au moins partiellement ladite ou lesdites parois latérales (3), ledit au moins un panneau souple (12) comportant au moins une partie transparente destinée à venir se positionner en regard du panneau transparent (261) de la partie supérieure (26) de ladite paroi frontale (2), et des moyens de fixation sur ladite paroi frontale (2) et/ou, le cas échéant, sur ladite ou lesdites parois latérales (3),
- au moins une structure souple de sas (13), adaptée pour venir recouvrir au moins partiellement la structure d'obturation souple (72) associée à ladite ouverture de sas (71) de ladite structure de sas (7), laquelle au moins une structure souple de sas (13) se présente sous la forme d'une poche souple munie d'une ouverture, laquelle poche souple est adaptée pour venir recouvrir ladite structure d'obturation souple (72) par engagement de la bordure inférieure de cette dernière dans ladite ouverture de ladite poche souple, laquelle structure souple de sas (13) est munie de moyens pour sa fixation sur ladite structure de sas (7), et
- une poche souple de dispositif support (14), pour le recouvrement dudit dispositif support (73) adapté au support de matériel(s) et/ou d'un bras de l'opérateur, laquelle poche souple de dispositif support (14) comprend une ouverture de poche pour son positionnement sur ledit dispositif support (73), laquelle poche souple de dispositif support (14) est munie de moyens pour sa fixation sur ledit dispositif support (73).

## Patentansprüche

1. Strahlenschutzschild (1) zum Sicherstellen des Schutzes eines Bedieners vor den Ausstrahlungen ionisierender Strahlen vom Typ der Röntgenstrahlen oder anderer Strahlen, wobei der Schutzschild (1) eine Vorderwand (2) aufweist, die mindestens teilweise aus einem Strahlenschutzmaterial gefertigt ist, wobei die Vorderwand (2) eine innere Vorderwandseite (24), die auf einen Positionierungsbereich (E) des Bedieners gerichtet ist, eine von der inneren Vorderwandseite (24) abgewandte äußere Vorderwandseite (25), einen oberen Teil (26), einen unteren Teil (27) und einen zwischen dem oberen und dem unteren Teil (26, 27) gelegenen Zwischenteil (28) aufweist, wobei der obere Teil (26) mindestens eine durchsichtige Platte (261) aufweist und wobei der Zwischenteil (28) eine Durchlaßstruktur (7) aufweist, die zum Hindurchführen von Material und/oder den Armen des Bedieners ausgelegt ist, wobei die Durchlaßstruktur (7) eine Durchlaßöffnung (71) aufweist, die einer flexiblen Verschlußstruktur (72) zugeordnet ist, wobei die Durchlaßöffnung (71) einen waagerechten oder im Wesentlichen waagerechten unteren Öffnungsrand (711) aufweist,
**dadurch gekennzeichnet, daß** der Schutzschild (1) eine Stützvorrichtung (73) aufweist, die zum Abstützen von Material und/oder eines Arms des Bedieners ausgelegt ist, wobei die Stützvorrichtung (73) im Bereich des unteren Öffnungsrands (711) auf der Seite der Innenseite (24) der Vorderwand angeordnet ist.

2. Strahlenschutzschild gemäß Anspruch 1, **dadurch gekennzeichnet, daß** die Stützvorrichtung (73) ein in der Nähe des unteren Öffnungsrands (711) gelegenes proximales Ende (731) und ein abgewandtes distales Ende (732) aufweist und daß das proximale Ende (731) der Stützvorrichtung (73) um eine senkrechte oder im Wesentlichen senkrechte Achse (733) schwenkbar montiert ist.

3. Strahlenschutzschild gemäß einem der Ansprüche 1 oder 2, **dadurch gekennzeichnet, daß** er Einstellmittel (74, 7111) aufweist, um ein Einstellen der Position der Stützvorrichtung (73) über mindestens einen Teil der Länge des unteren Öffnungsrands (711) zu ermöglichen.

4. Strahlenschutzschild gemäß Anspruch 3, **dadurch gekennzeichnet, daß** die Einstellmittel aus einer entlang des unteren Öffnungsrands (711) eingerichteten Schienenstruktur (7111) bestehen, die mit einer mit der Stützvorrichtung (73) fest verbundenen Gleitstückstruktur (74) zusammenwirken, wobei die Gleitstückstruktur (74) mit Verriegelungsmitteln versehen ist, die dazu ausgelegt sind, ein Verriegeln ihrer Position auf der Schienenstruktur (7111) zu ermöglichen.

5. Strahlenschutzschild gemäß einem der Ansprüche 1 bis 4, **dadurch gekennzeichnet, daß** die Stützvorrichtung (73) in Form eines länglichen Körpers vorliegt.

6. Strahlenschutzschild gemäß Anspruch 5, **dadurch gekennzeichnet, daß** die Stützvorrichtung (73) in Form einer Rinne mit U-förmigem Querschnitt vorliegt.

7. Strahlenschutzschild gemäß einem der Ansprüche 1 bis 6, **dadurch gekennzeichnet, daß** die Vorderwand (2) (a) eine äußere Platte (8), die mindestens einen Teil der Vorderwandaußenseite (25) bildet, wobei die äußere Platte (8) eine im Bereich des Zwischenteils (28) der Vorderwand gelegene Außenplattenöffnung (81) aufweist, und (b) eine senkrecht bewegliche innere Platte (9), die auf der Seite der Innenseite (24) der Vorderwand gegenüber der Außenplattenöffnung (81) angeordnet ist, aufweist,
wobei die innere Platte (9) eine Innenplattenöffnung (91) aufweist, wobei die Innenplattenöffnung (91) die flexible Verschlußstruktur (72) aufweist und den mit der Stützvorrichtung (73) versehenen unteren Öffnungsrand (711) aufweist,
wobei die Außenplattenöffnung (81) und die Innenplattenöffnung (91) gemeinsam die Durchlaßöffnung (71) bilden
und wobei die innere Platte (9) gegenüber der äußeren Platte (8) senkrecht bewegbar montiert ist, um zu ermöglichen, die Höhe der Durchlaßöffnung (71) einzustellen.

8. Strahlenschutzschild gemäß Anspruch 7, **dadurch gekennzeichnet, daß** die innere Platte (9) zusammen mit Gewichtsausgleichmitteln entlang auf der äußeren Platte (8) eingerichteter senkrechter Gleitschienen beweglich montiert ist, wobei die innere Platte (9) mit einem Betätigungsgriff (92) ausgestattet ist und einen Indexierungsfinger aufweist, der mit einer Anzahl auf der äußeren Platte (8) vorgesehener Indexierungslöcher zusammenwirkt, um die Verriegelung ihrer Höhenposition sicherzustellen.

9. Strahlenschutzschild gemäß einem der Ansprüche 7 oder 8, **dadurch gekennzeichnet, daß** die Innenplattenöffnung (91) und die Außenplattenöffnung (81) jeweils zwei waagerecht nebeneinander liegende und durch einen senkrechten Holm (83, 93) getrennte Öffnungsteile aufweisen.

10. Strahlenschutzschild gemäß einem der Ansprüche 1 bis 9, **dadurch gekennzeichnet, daß** die flexible Verschlußstruktur (72) aus einer Nebeneinanderanordnung einer Anzahl aus einem Strahlenschutzmaterial gefertigter senkrechter Bänder besteht.

11. Strahlenschutzschild gemäß einem der Ansprüche 1 bis 10, **dadurch gekennzeichnet, daß** sich die Vorderwand (2) in einer senkrechten oder im Wesentlichen senkrechten Ebene erstreckt und zwei Seitenränder (21) aufweist, wobei mindestens einer der Seitenränder (21) der Vorderwand (2) durch eine Seitenwand (3) verlängert wird, die sich, von der Innenseite (24) der Vorderwand her gesehen, in einer senkrechten oder im Wesentlichen senkrechten Ebene in einem Winkel (X) zwischen 90° und 140° zur Ebene der Vorderwand erstreckt,
wobei die Seitenwand oder die Seitenwände (3) einen mit einer flexiblen Schürze aus einem Strahlenschutzmaterial versehenen äußeren Seitenrand (31) aufweisen.

12. Strahlenschutzschild gemäß einem der Ansprüche 1 bis 11, **dadurch gekennzeichnet, daß** sich die Vorderwand (2) in einer senkrechten oder im Wesentlichen senkrechten Ebene erstreckt und zwei Seitenränder (21) aufweist, die beide durch eine Seitenwand (3) verlängert werden, wobei sich die Seitenwände (3), von der Innenseite (24) der Vorderwand her gesehen, jeweils in einer senkrechten oder im Wesentlichen senkrechten Ebene in einem Winkel (X) zwischen 90° und 140° zur Ebene der Vorderwand erstrecken,
und daß sich die Öffnungsstruktur (7) über die gesamte Breite der Vorderwand (2) erstreckt und über einen Teil der Breite jeder der beiden Seitenwände (3) verlängert wird.

13. Strahlenschutzschild gemäß einem der Ansprüche 1 bis 12, **dadurch gekennzeichnet, daß** er einen Schirm (10) aufweist, der auf einem Schirmgestell (11) befestigt ist, das an der Innenseite (24) der Vorderwand oder, gegebenenfalls, an einer Innenseite (34) einer der Seitenwände (3) befestigt ist.

14. Strahlenschutzschild gemäß einem der Ansprüche 1 bis 13, **dadurch gekennzeichnet, daß** er ein mit Bodenrädern (6) ausgestattetes Gestell (5) aufweist und daß der untere Teil (27) der Vorderwand (2) und, gegebenenfalls, ein unterer Teil der Seitenwände (3) eine Platte (271) aus flexiblem Strahlenschutzmaterial aufweist.

15. Ausstattung in Form einer Hülle, die dazu bestimmt ist, mindestens einen Teil der Höhe eines Strahlenschutzschilds (1) gemäß einem der Ansprüche 1 bis 14 zu bedecken, **dadurch gekennzeichnet, daß** sie
- mindestens eine flexible Platte (12), die dazu ausgelegt ist, die Vorderwand (2) mindestens teilweise und, gegebenenfalls, die Seitenwände (3) mindestens teilweise zu bedecken, wobei die mindestens eine flexible Platte (12) mindestens einen durchsichtigen Teil, der dazu bestimmt ist, gegenüber der durchsichtigen Platte (261) des oberen Teils (26) der Vorderwand (2) positioniert zu werden, und Mittel zum Befestigen an der Vorderwand (2) und/oder, gegebenenfalls, an der Seitenwand oder den Seitenwänden (3) aufweist,
- mindestens eine flexible Durchlaßstruktur (13), die dazu ausgelegt ist, die flexible der Durchlaßöffnung (71) der Durchlaßstruktur (7) zugeordnete Verschlußstruktur (72) mindestens teilweise zu bedecken, wobei die mindestens eine flexible Durchlaßstruktur (13) in Form einer mit einer Öffnung versehenen flexiblen Tasche vorliegt, wobei die flexible Tasche dazu ausgelegt ist, die flexible Verschlußstruktur (72) durch Eingreifen des unteren Rands der letzteren in die Öffnung der flexiblen Tasche zu bedecken, wobei die flexible Durchlaßstruktur (13) mit Mitteln für deren Befestigung an der Durchlaßstruktur (7) versehen ist, und
- eine flexible Tasche einer Tragevorrichtung (14) zum Bedecken der für das Abstützen von Material und/oder eines Arms des Bedieners ausgelegten Stützvorrichtung (73), wobei die flexible Tasche einer Tragevorrichtung (14) eine Taschenöffnung für deren Positionierung auf der Stützvorrichtung (73) aufweist, wobei die flexible Tasche einer Tragevorrichtung (14) mit Mitteln für deren Befestigung an der Stützvorrichtung (73) versehen ist,
aufweist.

## Claims

1. Radiation protection screen (1) for protecting an operator against the emission of ionizing radiation of the X-ray type or other, which screen (1) comprises a front wall (2) made at least in part from a radiation-protective material, which front wall (2) comprises a front wall inner face (24), facing an operator positioning space (E), a front wall outer face (25), opposite to said front wall inner face (24), an upper part (26), a lower part (27) and an intermediate part (28) located between said upper (26) and lower (27) parts, which upper part (26) comprises at least one transparent panel (261) and which intermediate part (28) comprises an airlock structure (7), suitable for the passage of material(s) and/or the arms of said operator,
which airlock structure (7) comprises an airlock opening (71) associated with a flexible closing structure (72), which airlock opening (71) comprises a lower opening edge (711), horizontal or approximately horizontal,
**characterized in that** said screen (1) comprises a support device (73) suitable for supporting material(s) and/or an operator's arm, which support device (73) is arranged at the said lower edge opening (711), on the side of said inner face of the front wall (24).

2. Radiation protection screen according to claim 1, **characterized in that** said support device (73) comprises a proximal end (731) located close to said lower edge of the opening (711) and an opposite distal end (732), and **in that** said proximal end (731) of said support device (73) is pivotally mounted about a vertical or approximately vertical axis (733).

3. Radiation protection screen according to any one of claims 1 or 2, **characterized in that** it comprises adjustment means (74, 7111) to allow adjustment of the position of said support device (73) over at least part of the length of said lower opening edge (711).

4. Radiation protection screen according to claim 3, **characterized in that** said adjustment means consist of a rail structure (7111) formed along said lower edge of the opening (711), cooperating with a slider structure (74) integral with said support device (73), which slider structure (74) is provided with locking means adapted to allow locking of its position on said rail structure (7111).

5. Radiation protection screen according to any one of claims 1 to 4, **characterized in that** said support device (73) is in the form of an elongated body.

6. Radiation protection screen according to claim 5, **characterized in that** said support device (73) is in the form of a chute with a U-section.

7. Radiation protection screen according to any one of claims 1 to 6, **characterized in that** said front wall (2) comprises (a) an outer panel (8), forming at least part of said outer face of the front wall (25), which outer panel (8) comprises an outer panel opening (81) located at the intermediate part of the front wall (28), and (b) an inner panel (9), movable vertically, arranged facing said opening of outer panel (81), on the side of said inner face of front wall (24),
which inner panel (9) comprises an inner panel opening (91), which inner panel opening (91) comprises said flexible closing structure (72) and comprises said lower opening edge (711) equipped with said support device (73),
which outer panel opening (81) and which inner panel opening (91) together form said airlock opening (71),
and which inner panel (9) is movably mounted vertically relative to said outer panel (8) so as to allow the height level of said airlock opening (71) to be adjusted.

8. Radiation protection screen according to claim 7, **characterized in that** said inner panel (9) is movably mounted along vertical rails (82) made on the said outer panel (8), in association with weight compensating means, which inner panel (9) is provided with an maneuvering handle (92) and comprises an indexing finger cooperating with a plurality of indexing holes provided on said outer panel (8), to ensure the locking of its height position.

9. Radiation protection screen according to any one of claims 7 or 8, **characterized in that** the said interior panel opening (91) and the said exterior panel opening (81) each comprise two opening parts juxtaposed horizontally and separated by a vertical upright. (83, 93).

10. Radiation protection screen according to any one of claims 1 to 9, **characterized in that** the said flexible closing structure (72) consists of a juxtaposition of a plurality of vertical strips made of radio-protective material.

11. Radiation protection screen according to any one of claims 1 to 10, **characterized in that** the said front wall (2) extends in a vertical or approximately vertical plane and comprises two side edges (21), at least one of the said side edges (21) of said front wall (2) extending by a side wall (3) which extends in a vertical or approximately vertical plane, at an angle (X) of between 90° and 140° with respect to the plane of said front wall, seen from the side of said inner face of the front wall (24),
which side wall(s) (3) comprise an outer side edge (31) fitted with a flexible flap (36) made of radio-protective material.

12. Radiation protection screen according to any one of claims 1 to 11, **characterized in that** said front wall (2) extends in a vertical or approximately vertical plane and comprises two side edges (21) each extending by a side wall (3), which side walls (3) each extend in a vertical or approximately vertical plane, at an angle (X) comprised between 90° and 140° with respect to the plane of said front wall (2), seen from the side of said front wall inner face (24),
and **in that** said airlock structure (7) extends over the entire width of said front wall (2) and extends over part of the width of each of said side walls (3).

13. Radiation protection screen according to any one of claims 1 to 12, **characterized in that** it comprises a screen (10) mounted on a screen support (11) fixed to said internal face of the front wall (24) or, where applicable, on an inner face (34) of one of its side walls (3).

14. Radiation protection screen according to any one of claims 1 to 13, **characterized in that** it comprises a base (5) provided with ground support wheels (6), and **in that** the lower part (27) of its front wall (2) and, where applicable, a lower part of its side walls (3), comprises a panel of flexible radio-protective material (271).

15. Equipment in the form of a cover intended to cover at least part of the height of a radiation protection screen (1) according to any one of claims 1 to 14, **characterized in that** it comprises:
- at least one flexible panel (12) adapted to at least partially cover said front wall (2) and if necessary at least partially said side wall(s) (3), said at least one flexible panel (12) comprising at least a transparent part intended to be positioned opposite the transparent panel (261) of the upper part (26) of said front wall (2), and fixing means on said front wall (2) and/or, where applicable, on said side wall(s) (3),
- at least one flexible airlock structure (13), adapted to at least partially cover the flexible closing structure (72) associated with said airlock opening (71) of said airlock structure (7), which at least one flexible airlock structure (13) is in the form of a flexible pocket provided with an opening, which flexible pocket is adapted to cover said flexible closing structure (72) by engagement of the lower edge of the latter in said opening of said flexible pocket, which flexible airlock structure (13) is provided with means for its attachment to said airlock structure (7), and
- a flexible support device pocket (14), for covering said support device (73) suitable for supporting material(s) and/or an operator's arm, which flexible support device pocket (14) comprises a pocket opening for its positioning on said support device (73), which flexible support device pocket (14) is provided with means for its attachment to said support device (73).
